(19) Europäisches Patentamt
European Patent Office
Office européen des brevets

(11) Publication number: **0 173 059**
**B1**

(12) **EUROPEAN PATENT SPECIFICATION**

(45) Date of publication of patent specification: **29.11.89**

(51) Int. Cl.$^4$: **C 07 H 19/173**

(21) Application number: **85108955.7**

(22) Date of filing: **17.07.85**

(54) **Method for the production of 2'-deoxyadenosine compounds.**

(30) Priority: **06.08.84 US 638192**

(43) Date of publication of application:
**05.03.86 Bulletin 86/10**

(45) Publication of the grant of the patent:
**29.11.89 Bulletin 89/48**

(84) Designated Contracting States:
**AT BE CH DE FR GB IT LI LU NL SE**

(56) References cited:
**US-A-3 225 029**
**US-A-3 708 469**

**NUCLEIC ACIDS RESEARCH, vol. 12, no. 2, February 1984, pages 1179-1192, IRL Press LTD, Oxford, GB; Z.KAZIMIERCZUK et al.: "Total synthesis of certain 2-, 6-mono- and 2,6-disubstituted-tubercidin derivatives. Synthesis of tubercidin via the sodium salt glycosylation procedure"**

**The file contains technical information submitted after the application was filed and not included in this specification**

(73) Proprietor: **BRIGHAM YOUNG UNIVERSITY**
**C-37 ASB**
**Provo Utah 84602 (US)**

(72) Inventor: **Robins, Roland Kenith**
**4006 Sherwood**
**Provo Utah 84604 (US)**
Inventor: **Revankar, Ganapathi Ramakrishna**
**1003 North 560 East Street**
**Orem Utah 84057 (US)**

(74) Representative: **Schaumburg, Thoenes & Englaender**
**Mauerkircherstrasse 31 Postfach 86 07 48**
**D-8000 München 80 (DE)**

(56) References cited:
**LIEBIGS ANNALEN DER CHEMIE, 1983, pages 876-884, Verlag Chemie GmbH, Weinheim, DE; F.SEELA et al.: "2'-Desoxytubercidin - Synthese eines 2'-Desoxyadenosin-Isosteren durch Phasentransferglycosylierung"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 173 059 B1**

Courier Press, Leamington Spa, England.

(56) References cited:
JOURNAL OF THE AMERICAN CHEMICAL SOCIETY, vol. 106, no. 21, 1984, pages 6379-6382, American Chemical Society, Columbus, Ohio, US; Z.KAZIMIERCZUK et al.: "Synthesis of 2'-Deoxytubercidin, 2'-Deoxyadenosine, and related 2'-Deoxynucleosides via a novel direct stereospecific sodium salt glycosylation procedure"

**Description**

Technical Field

This invention relates to a method for the production of 2'-deoxyadenosine compounds and related analogs, comprising a direct stereospecific glycosylation of a purine aglycon.

Background of the Invention

Prior glycosylation procedures in which the 2-deoxy-β-*D*-ribofuranosyl (2-deoxy-β-*D*-*erythro*-pentofuranosyl moiety is introduced into an aglycon invariably provide anomeric mixtures as well as positional isomers which result in very low yields of the desired 2'-deoxynucleoside. In view of these difficulties, a four-step deoxygenation procedure using phenoxythiocarbonylation (J.A.C.S. 1983, *105*, 4059) or imidazolylthiocarbonylation (J. Org. Chem. 1982, *47*, 485; Chem. Pharm. Bull. 1983, *31*, 1842) of the 2'-hydroxy group of the corresponding 3',5'-protected β-*D*-ribonucleoside has been developed to provide the requisite 2'-deoxynucleoside. What the art lacks, however, are improved procedures that do not require the availability of the preformed ribonucleoside and also are applicable in the presence of haloheterocylic derivatives, which are the most useful for further nucleophilic displacement.

F. Seela and A. Kehne, Liebigs Ann. Chemie 1983, 867—884, describe the synthesis of 2'-deoxytubercidin, an isoster of 2'-deoxyadenosine, by phase-transfer glycosylation under aqueous biphasic conditions in the presence of a phase-transfer catalyst.

Summary and Detailed Description

The present invention, which overcomes the difficulties and shortcomings of the prior art, concerns a method of producing 2'-deoxyadenosine compounds, comprising glycosylating a sodium salt of a purine compound having the formula

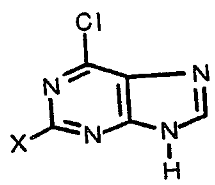

with 1-chloro-2-deoxy-3,5-di-*O*-*p*-toluoyl-α-*D*-*erythro*-pentofuranose in a polar solvent, isolating the resulting 9-(2-deoxy-3,5-di-*O*-*p*-toluoyl-β-*D*-*erythro*-pentofuranosyl) purine having the formula I

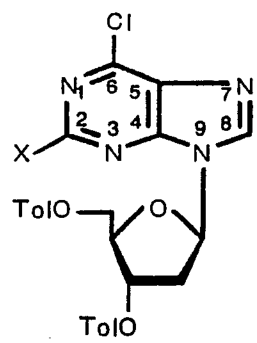

I

and subjecting the same to ammonolysis under deblocking conditions to obtain the corresponding 2'-deoxyadenosine having the formula II

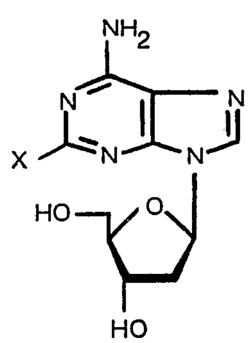

II

where X is hydrogen or Cl.

The glycosylation step of the method is carried out in a polar solvent, preferably acetonitrile, at ambient temperature. The sodium salt of the purine compound preferably is formed in situ by reaction of the purine compound and sodium hydride. The reaction is usually complete within 0.5 hour. The glycosylation is carried out at ambient temperature until complete, usually within about 15 hours. The specificity of the glycosylation step and the high yield produced of the desired 6-chloro-9-glycosylated product were both unexpected. Although a polar solvent was used, which would tend to favor sugar anomerization and thus resulting in approximately equal amounts of the alpha and beta nucleosidic products, the desired β-glycosylation was rapid and preferentially took place without appreciable anomerization. Since the starting 1-chloro-2-deoxy-3,5-di-*O*-*p*-toluoyl-*D*-*erythro*-pentofuranose has the α-configuration in the solid state, the exclusive formation of the blocked 2′-deoxy-β-nucleosides is viewed to be due to a direct Walden inversion ($S_N2$) at the $C_1$ carbon by the anionic heterocyclic nitrogen. Inasmuch as the reaction mixture may contain a minor amount of positional isomers, the product is isolated from the reaction mixture in any suitable way, preferably by column chromatography. The ammonolysis is conveniently carried out by heating a solution of the 6-chloro-9-pentafuranosylpurine product in methanolic ammonia at elevated temperature, preferably about 100°C, until the reaction is complete, usually for a period of from about 5 to about 12 hours (cf. Z. Kazimierczank et al., Nucleic Acids Research 12, 1984, 1179—1192). The products of the method, 2′-deoxyadenosine and 2-chloro-2′-deoxyadenosine, are useful cytotoxic agents and are useful as intermediates for the production of 2′-deoxyadenosine analogs (Cf. Proc. Am. Assoc. Cancer Res. 1980, *71*, 302; Cancer Res. 1982, *42*, 3911).

The invention is illustrated, and the best mode of carrying out the same is set forth, in the following examples.

General Procedures

Melting points were taken on Thomas-Hoover capillary melting point apparatus and are uncorrected. Nuclear magnetic resonance ($^1H$ NMR) spectra were determined at 90 MHz with a JEOL FX 900 spectrometer. The chemical-shift values are expressed in delta values (parts per million) relative to tetramethylsilane as an internal standard. Ultraviolet spectra (UV; sh = shoulder) were recorded on a Cary Model 15 spectrophotometer. Evaporations were carried out under reduced pressure with the bath temperature below 30°C.

Example 1

2,6-Dichloro-9-(2-deoxy-3,5-di-*O*-*p*-toluoyl-β-*D*-*erythro*-pentofuranosyl)-purine

a) A mixture of 2,6-dichloropurine (0.95 g, 5 mmol) and sodium hydride (50% in oil, 0.25 g, 5.2 mmol) in anhydrous $CH_3CN$ (35 mL) was stirred at ambient temperature under a nitrogen atmosphere for 30 min. Dry, powdered 1-chloro-2-deoxy-3,5-di-*O*-*p*-toluoyl-α-*D*-*erythro*-pentafuranose (1.95 g, 5 mmol) was added portionwise with stirring, during 20 min, and stirring was continued for a further 15 h. A small amount of insoluble material was removed by filtration. Evaporation of the solvent gave an oily residue, which was purified on a silica gel column (5 × 60 cm) using toluene:acetone (9:1, v/v) as the solvent. Two nucleosides were isolated in the order listed: the title compound was crystallized from EtOH to yield 1.60 g (50%); mp 159—162°C [Literature mp 155—157°C].

b) The N-7 glycosyl isomer 2,6-dichloro-7-(2-deoxy-3,5-di-*O*-*p*-toluoyl-β-*D*-*erythro*-pentofuranosyl) purine was isolated and crystallized from EtOH to yield 0.35 g (13%); mp 141—143°C. $^1H$ NMR ($Me_2SO$-$d_6$ δ 6.88 (t, 1, $C_1$, *H*, peak width 14.5 Hz), 7.36 and 7.90 (m, 8, Ph), 9.28 (s, 1, $C_8H$). Anal. Calcd for $C_{26}H_{22}Cl_2N_4O_5$ (541.4): C, 57.68; H, 4.09; N, 10.35. Found: C, 57.55; H, 4.00; N, 10.36.

2-Chloro-6-amino-9-(2-deoxy-β-*D*-*erythro*-pentofuranosyl)purine (2-Chloro-2′-deoxyadenosine)

c) A solution of the 2,6-dichloro-9-pentofuranosylpurine product of 1a) (2.50 g, 4.6 mmol) in $CH_3OH$/$NH_3$ (saturated at 0°C, 60 ml) was heated at 100°C for 5 h and the mixture was evaporated to dryness. The residue was purified on a silica gel column (5 × 40 cm) using $CHCl_3$:MeOH (8:2, v/v) as the solvent. Crystallization of the homogeneous solid from EtOH gave 0.87 g (71% of analytically pure title compound; mp 220°C (softens), resolidifies, turns brown, does not melt below 300°C [Lit. mp 210—215°C (softens) and then solidifies and turns brown].

Example 2

6-Chloro-9-(2-deoxy-3,5-di-*O*-*p*-toluoyl-β-*D*-*erythro*-pentofuranosyl)purine

a) In the same manner as for Example 1, reaction of the sodium salt of 6-chloropurine (J. Med. Chem. *1984*, 27, 175, 0.77 g, 5 mmol and 50% NaH in oil, 0.25 g, 5.2 mmol) with 1-chloro-2-deoxy-3,5-di-*O*-*p*-toluoyl-α-*D*-*erythro*-pentafuranose (2.0 g, 5.15 mmol) in $CH_3CN$ (50 mL) gave 1.51 g (59%) of crystalline title compound (from EtOH); mp 107—109°C. $^1H$ NMR ($Me_2SO$-$d_6$) δ 6.76 (t, 1, $C_1$, *H*, peak width 14.0 Hz), 7.36 and 7.94 (m, 8, Ph), 8.80 (s, 1, $C_2H$), 9.00 (s, 1, $C_1H$). Anal. Calcd. for $C_{26}H_{23}ClN_4O_5$ (506.9): C, 61.60; H, 4.57; N, 11.05. Found: C, 61.73; H, 4.72; N, 11.03.

b) The N-7 glycosyl isomer 6-chloro-7-(2-deoxy-3,5-di-*O*-*p*-toluoyl-β-*D*-*erythro*-pentofuranosyl)purine was isolated and crystallized from EtOH to yield 0.29 g (11%); mp 152—153°C. $^1H$ NMR ($Me_2SO$-$d_6$) δ 6.96 (t, 1, $C_1$, *H*, peak width 14.5 Hz), 7.36 and 7.94 (m, 8, Ph), 8.94 (s, 1, $C_2H$), 9.26 (s, 1, $C_8H$). Anal. Calcd for $C_{26}H_{23}ClN_4O_5$ (506.9): C, 61.60; H, 4.57; N, 11.05. Found: C, 61.55; H, 4.49; N, 11.05.

6-Amino-9-(2-deoxy-β-*D-erythro*-pentofuranosyl)pyrine (2′-Deoxyadenosine)

c) A solution of the 6-chloro-9-pentofuranosylpurine product of 2a) (1.01 g, 2 mmol) in $MeOH/NH_3$ (18 mL) was heated at 100°C for 12 h and then evaporated to dryness. The aqueous solution of the residue was extracted with $CHCl_3$ (2 × 25 mL), followed by ether (2 × 25 mL) and then evaporated to dryness. The residue was crystallized from water to yield 0.41 g (78%); mp 186—189°C [Lit. mp 187—189°C, and all other physicochemical properties of the title 2′-deoxyadenosine product are identical with 2′-deoxyadenosine reported in the literature].

The embodiments of the invention in which an exclusive property or privilege is claimed are defined as follows:

**Claims**

1. A method of producing 2′-deoxyadenosine compounds, comprising glycosylating a sodium salt of a purine compound having the formula

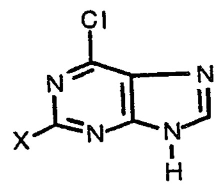

with 1-chloro-2-deoxy-3,5-di-*O-p*-toluoyl-α-*D-erythro*-pentofuranose in a polar solvent, isolating the resulting 9-(2-deoxy-3,5-di-*O-p*-toluoyl-β-*D-erythro*-pentofuranosyl) purine having the formula I

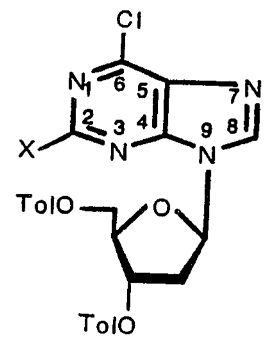

I

and subjecting the same to ammonolysis under deblocking conditions to obtain the corresponding 2′-deoxyadenosine having the formula II

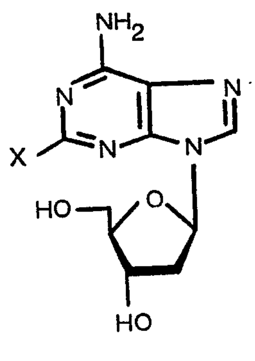

II

where X is hydrogen or Cl.

2. A method according to Claim 1 where acetonitrile is employed as the polar solvent in the glycosylation step.

3. A method according to Claim 1 or 2 where the sodium salt is formed in situ at ambient temperature by reaction of the purine compound and sodium hydride.

4. A method according to any of the preceding claims where the ammonolysis is carried out by heating a solution of the 6-chloro-9-pentofuranosylpurine product in methanolic ammonia at elevated temperature.

5. A method according to any of the preceding claims where the purine compound is 2,6-dichloropurine.

6. A method according to any of the preceding claims where the purine compound is 6-chloropurine.
7. 2,6-Dichloro-9-(2-deoxy-3,5-di-*O-p*-toluoyl-β-*D-erythro*-pentofuranosyl)purine.
8. 6-Chloro-9-(2-deoxy-3,5-di-*O-p*-toluoyl-β-*D-erythro*-pentofuranosyl)purine.
9. 2-Chloro-2′-deoxyadenosine produced by the method according to Claim 1.
10. 2′-Deoxyadenosine produced by the method according to Claim 1.

**Patentansprüche**

1. Verfahren zur Herstellung von 2′-Deoxyadenosin-Verbindungen, mit den Stufen: Glycosylierung eines Natriumsalzes einer Purin-Verbindung der Formel

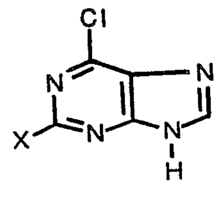

mit 1-Chlor-2-deoxy-3,5-di-o-p-toluoyl-α-*D-erythro*-pentofuranose in einem polaren Lösungsmittel, Isolierung des sich bildenden 9-(2-Deoxy-3,5-di-o-p-toluoyl-β-*D-erythro*-pentofuranosyl)-purins mit der Formel I

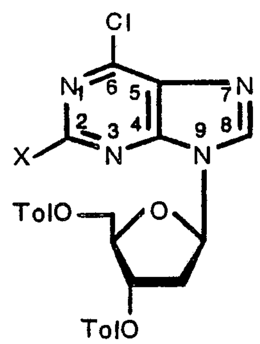

I

und Ammonolyse dieser Verbindung unter deblockierenden Bedingungen, um das entsprechende 2′-Deoxyadenosin mit der Formel II

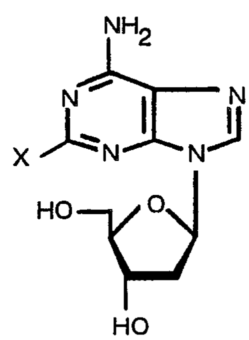

II

zu erhalten, worin X Wasserstoff oder Cl ist.

2. Verfahren nach Anspruch 1, dadurch gekennzeichnet, daß als polares Lösungsmittel in der Glycosylierungsstufe Acetonitril verwendet wird.

3. Verfahren nach Anspruch 1 oder 2, dadurch gekennzeichnet, daß das Natriumsalz in situ bei Raumtemperatur durch Reaktion der Purin-Verbindung und Natriumhydrid gebildet wird.

4. Verfahren nach einem der vorhergehenden Ansprüche, dadurch gekennzeichnet, daß die Ammonolyse ausgeführt wird durch Erhitzen einer Lösung des 6-Chlor-9-pentofuranosyl-purin-Produktes in methanolischem Ammoniak bei erhöhter Temperatur.

5. Verfahren nach einem der vorhergehenden Ansprüche, dadurch gekennzeichnet, daß die Purin-Verbindung 2,6-Dichlorpurin ist.

6. Verfahren nach einem der vorhergehenden Ansprüche, dadurch gekennzeichnet, daß die Purin-Verbindung 6-Chlorpurin ist.

7. 2,6-Dichlor-9-(2-deoxy-3,5-di-o-p-toluoyl-β-*D-erythro*-pentofuranosyl)-purin.

8. 6-Chlor-9-(2-deoxy-3,5-di-o-p-toluoyl-β-*D-erythro*-pentofuranosyl)-purin.

9. 2-Chlor-2′-deoxyadenosin, hergestellt nach dem Verfahren gemäß Anspruch 1.

10. 2′-Deoxyadenosin, hergestellt nach dem Verfahren gemäß Anspruch 1.

**Revendications**

1. Procédé pour la préparation de composés 2′-désoxyadénosine comprenant la glycosylation d'un sel de sodium d'un composé de purine ayant la formule

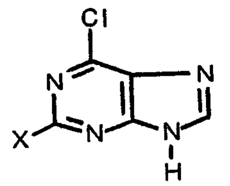

avec un 1-chloro-2-désoxy-3,5-di-*O-p*-toluyl-α-*D-érythro*-pentofuranose dans un solvant polaire, l'isolement de la 9-(2-désoxy-3,5-di-*O-p*-toluyl-β-D-*érythro*-pentofuranosyl)-purine ayant la formule I

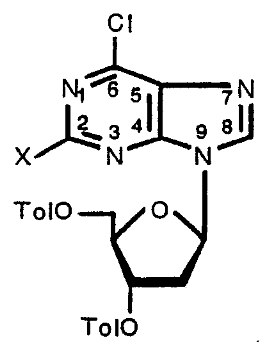

I

et la soumission de celle-ci à une ammoniolyse dans des conditions débloquantes pour l'obtention de la 2′-désoxyadénosine correspondante ayant la formule II

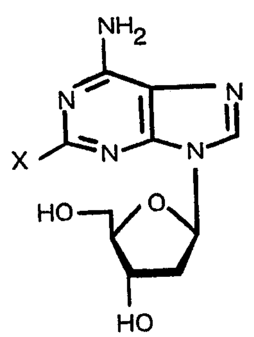

II

dans laquelle X est l'hydrogène ou Cl.

2. Procédé selon la revendication 1 selon lequel l'acétonitrile est employé comme solvant polaire à l'opération de glycosylation.

3. Procédé selon la revendication 1 ou 2 selon lequel le sel de sodium est formé in situ à la température du milieu ambiant par réaction du composé de purine et de l'hydrure de sodium.

4. Procédé selon l'une quelconque des revendications précédentes selon lequel l'ammoniolyse est effectuée par chauffage d'une solution du produit 6-chloro-9-pentofuranosylpurine dans de l'ammoniac méthanolique à température élevée.

5. Procédé selon l'une quelconque des revendications précédentes selon lequel la purine est la 2,6-dichloropurine.

6. Procédé selon l'une quelconque des revendications précédentes selon lequel la purine est la 6-chloropurine.

7. 2,6-dichloro-9-(2-désoxy-3,5-di-*O-p*-toluyl-β-*D-érythro*-pentofuranosyl)purine.
8. 6-chloro-9-(2-désoxy-3,5-di-*O-p*-toluyl-β-*D-érythro*-pentofuranosyl)purine.
9. 2-chloro-2'désoxyadénosine produite par le procédé de la revendication 1.
10. 2'-désoxyadénosine produite par le procédé de la revendication 1.